# EUROPÄISCHE PATENTSCHRIFT

(11) **EP 4 518 930 B1**
(45) Veröffentlichungstag und Bekanntmachung des Hinweises auf die Patenterteilung: **04.02.2026**
(21) Anmeldenummer: 23725136.8
(22) Anmeldetag: 03.05.2023
(51) Int. Cl.: A61M 5/32

(54) **SCHUTZVORRICHTUNG FÜR EIN NADELROHR EINER SPRITZE**
PROTECTION DEVICE FOR A NEEDLE TUBE OF A SYRINGE
DISPOSITIF DE PROTECTION POUR UN TUBE D'AIGUILLE D'UNE SERINGUE

(30) Priorität: 05.05.2022 DE 102022111083
(43) Veröffentlichungstag der Anmeldung: 12.03.2025
(73) Patentinhaber: SAFEJECT INTERNATIONAL PTE LTD, Singapore 079903 (SG)
(72) Erfinder: FISCHER, Stephan, 32120 Hiddenhausen (DE); MOHR, Bernd, 25355 Barmstedt (DE); WILKE, Tobias, 49477 Ibbenbüren (DE)
(74) Vertreter: Bauer PSU PartG mbB
(86) Internationale Anmeldenummer: PCT/EP2023/061677
(87) Internationale Veröffentlichungsnummer: WO 2023/213883

(56) Entgegenhaltungen:
- DE-A1- 102017 120 538
- DE-U1- 202019 103 876
- US-A- 5 490 841
- US-A1- 2020 078 532
- US-B2- 11 471 611

## Beschreibung

### Beschreibung

Die vorliegende Anmeldung betrifft eine Schutzvorrichtung für ein Nadelrohr einer Spritze gemäß dem Oberbegriff von Anspruch 1. Ferner betrifft die vorliegende Anmeldung ein Set umfassend eine Schutzvorrichtung sowie einen Nadelträger mit einem darin gelagerten Nadelrohr.

Die Schutzvorrichtung umfasst eine Basis sowie eine mit der Basis verbundene Abdeckung. Die Abdeckung ist relativ zu der Basis verschwenkbar, wobei typischerweise die Basis und die Abdeckung mittels eines Folienscharniers miteinander verbunden sind. Die Basis ist dazu eingerichtet, mit einem Nadelträger formschlüssig zusammenzuwirken. Der Nadelträger ist dazu eingerichtet, das Nadelrohr zu tragen. Hierbei ist das Nadelrohr typischerweise zentral in eine Ausnehmung des Nadelträgers eingeklebt, sodass eine Kraft übertragende Verbindung zwischen dem Nadelrohr und dem Nadelträger gebildet ist. Infolge der formschlüssigen Verbindung der Basis mit dem Nadelträger ist die Basis in der Regel fest mit dem Nadelträger verbunden.

Die Abdeckung ist langgestreckt ausgebildet und erstreckt sich entlang einer Längsachse. Die Abdeckung begrenzt einen Innenraum, in dem das Nadelrohr zum Schutz einer Person vor einem unbeabsichtigten Kontakt mit dem Nadelrohr lagerbar ist. Insbesondere sollen Personen davor geschützt werden, mit einer Nadelspitze des Nadelrohrs unbeabsichtigten Kontakt zu treten, vor allem nach einem Injektionsgebrauch des Nadelrohrs. Auf diese Weise kann verhindert werden, dass Infektionskrankheiten von einem Patienten, der mit dem Nadelrohr gestochen wurde, auf eine den Patienten behandelnde Person übertragen werden.

Die Schwenkbarkeit der Abdeckung relativ zu der Basis ist in solcher Weise gegeben, dass die Abdeckung innerhalb einer Schwenkebene relativ zu der Basis bewegbar ist. Auf diese Weise kann die Abdeckung ausgehend von einer Anfangsstellung, in der das Nadelrohr vor einem Injektionsgebrauch in dem Innenraum der Abdeckung gelagert ist, relativ zu der Basis in eine erste Schwenkrichtung in eine Nutzstellung überführt werden. In der Nutzstellung ist das Nadelrohr zum Injektionsgebrauch freigegeben, das heißt es befindet sich nicht länger innerhalb des Innenraums der Abdeckung. Nach dem Injektionsgebrauch kann die Abdeckung in eine der ersten Schwenkrichtung entgegengesetzte zweite Schwenkrichtung ausgehend von der Nutzstellung zurück in eine Schutzstellung überführt werden, in der das Nadelrohr nach dem Injektionsgebrauch wieder in dem Innenraum gelagert ist. Hierdurch ist das Nadelrohr nach dem Injektionsgebrauch in der vorstehend beschriebenen Weise vor einem unbeabsichtigten Kontakt mit einer Person geschützt.

Damit das Nadelrohr bei der Überführung der Abdeckung von der Anfangsstellung in die Nutzstellung zunächst aus der Abdeckung austreten und schließlich bei Rücküberführung der Abdeckung von der Nutzstellung in die Schutzstellung wieder in die Abdeckung eintreten kann, ist die Abdeckung in der Schwenkebene an einer Seite offen. Insbesondere kann eine Wandung der Abdeckung, die den Innenraum der Abdeckung räumlich einfasst, den Innenraum lediglich an drei Seiten umschließen, während die vierte Seite offen ist.

Die Abdeckung weist in ihrem Innenraum eine Halteeinrichtung auf, mittels der das Nadelrohr im Zuge der Überführung der Abdeckung in die Schutzstellung formschlüssig verrastbar ist. Dies erfolgt in der Art, dass nach erfolgter Überführung der Abdeckung in die Schutzstellung ein unbeabsichtigtes Austreten des Nadelrohrs aus dem Innenraum unterbunden ist. Mit anderen Worten dient die Halteeinrichtung dazu, dass Nadelrohr "zurückzuhalten", sodass ein abermaliges Verschwenken der Abdeckung relativ zu der Basis nach dem Injektionsgebrauch des Nadelrohrs nicht dazu führt, dass das Nadelrohr freigegeben wird.

### Stand der Technik

Eine Schutzvorrichtung der eingangs beschriebenen Art ist im Stand der Technik bereits bekannt. Hierzu wird zunächst auf die internationale Patentanmeldung WO 2021/008717 A1 hingewiesen. Diese beschreibt eine Schutzvorrichtung, deren Abdeckung eine Halteeinrichtung zur sicheren Halterung des Nadelrohrs aufweist. Die Halteeinrichtung umfasst zwei Laschen, die sich gegenüberliegend voneinander jeweils ausgehend von einer inneren Mantelfläche einer Wandung der Abdeckung aufeinander zu erstrecken. Hierbei sind die Laschen bezogen auf die Schwenkebene geneigt ausgebildet sowie derart elastisch, dass das Nadelrohr die Laschen verformen, hierdurch verdrängen und auf diese Weise hinter den Laschen einrasten kann. Auf diese Weise wird sichergestellt, dass das Nadelrohr bei einer Verschwenkung der Abdeckung relativ zu der Basis der Schutzvorrichtung nicht aus dem hinter den Laschen aufgespannten Sicherungsbereich austreten kann, sodass eine unbeabsichtigte Verletzung einer Person durch das Nadelrohr unterbunden ist.

Wenngleich die Sicherheit der bekannten Schutzvorrichtung gegen ein unbeabsichtigtes Austreten des Nadelrohrs aus dem Sicherungsbereich bereits sehr groß ist, sind doch mutwillige Fehlbedienungen der Schutzvorrichtung denkbar, die dazu führen, dass das Nadelrohr nach seinem Injektionsgebrauch aus dem Sicherungsbereich austritt und daraufhin eine Infektionsgefahr darstellt.

Weiterhin wird auf das US-Patent 5,490,841 B1 hingewiesen, das ebenfalls eine Schutzvorrichtung mit einer Abdeckung offenbart. Die Abdeckung umfasst eine Halteeinrichtung, die wiederum eine schräg innerhalb eines Innenraums der Abdeckung orientierte Lasche umfasst. Die Lasche dient dazu, ein Nadelrohr nach dessen Injektionsgebrauch sicher in einem Sicherungsbereich des Innenraums der Abdeckung "einzuschließen", wobei die Lasche derart flexibel ausgebildet ist, dass sie zwecks Eintritts des Nadelrohrs in den Sicherungsbereich zunächst elastisch verformt werden kann. Eine Verformung in die Gegenrichtung, die zu einem Austritt des Nadelrohrs aus dem Sicherungsbereich führen könnte, ist mittels eines Anschlags verhindert, der an einer der Lasche gegenüberliegenden Stelle einer inneren Mantelfläche der Wandung der Abdeckung ausgebildet ist.

Die beschriebene Ausgestaltung ist insoweit nachteilig, als sich die Lasche in solcher Weise innerhalb des Innenraums der Abdeckung erstreckt, dass eine Kollision der Lasche mit dem Nadelrohr vor dessen Injektionsgebrauch auftreten kann. Dies ist insbesondere im Hinblick auf eine automatisierte Fertigung einer Spritze, die mit einem Nadelträger mitsamt einen Nadelrohr sowie einer Schutzvorrichtung ausgestattet ist, nachteilig, da die Schutzvorrichtung nicht ohne Weiteres mit ihrer Basis auf bzw. an dem Nadelträger montiert werden kann, ohne dass eine Kollision des in dem Nadelträger getragene Nadelrohrs mit der in dem Innenraum der Abdeckung befindlichen Lasche auftritt.

Die Druckschrift US 5,490,841 A offenbart eine Nadelschutzvorrichtung mit einem länglichen Gehäuse, das einen länglichen Nadeleingangsbereich aufweist, der zwischen zwei Gehäuseseitenwänden definiert ist. Es ist eine schwenkbare Falltür vorgesehen, die sich über den Eingangsbereich erstreckt, um die Nadel im Gehäuse einzuschließen. Die Falltür ist mittels eines elastischen Filmscharniers an einem Ende einer Seitenwand befestigt und erstreckt sich quer über den Eingangsbereich, um an einem Türanschlag an der gegenüberliegenden Seitenwand anzuliegen, so dass sich die Falltür um das Filmscharnier herum in Richtung der ersten Seitenwand drehen kann, um das Einführen der Nadel in das Gehäuse zu ermöglichen, aber durch den Türanschlag in dem Grad der Drehung von der ersten Seitenwand weg begrenzt ist, wodurch verhindert wird, dass eine Nadel, die sich bereits im Gehäuse befindet, durch den Eingangsbereich austreten kann.

Ferner offenbart die Druckschrift DE 10 2017 120 538 A1 offenbart eine Schutzvorrichtung zur Sicherung einer Nadel. Die Schutzvorrichtung umfasst ein Aufnahmeelement mit einem Aufnahmebereich zur Aufnahme der Nadel. Das Aufnahmeelement weist eine eine Abdeckung des Aufnahmebereichs bildende Abdeckeinrichtung auf. Die Abdeckeinrichtung weist eine Eintrittsöffnung für die Nadel zum Eindringen in den Aufnahmebereich auf. Die Abdeckeinrichtung ist derart beweglich an einem Seitenteil des Aufnahmeelements befestigt, dass sich die Eintrittsöffnung beim Drücken der Nadel auf die Abdeckeinrichtung vergrößert, so dass die Nadel in den Aufnahmebereich eindringt, und sich die Eintrittsöffnung wieder verkleinert, nachdem die Nadel in den Aufnahmebereich gelangt ist und kein Druck mehr auf die mindestens eine Abdeckeinrichtung ausgeübt wird.

Ferner offenbart die Druckschrift US 2020/0078532 A1 eine Vorrichtung zur Vermeidung von Nadelstichen für eine Injektionsnadel, die von einem nadeltragenden Element einer Spritze getragen wird. Sie ist als einteiliges Formteil ausgebildet und umfasst einen ersten Teil, der an dem nadeltragenden Element angebracht werden kann, und einen zweiten Teil, der einen Schutz für die Nadel bietet und relativ zu dem ersten Teil schwenkbar ist, um die Nadel zur Verwendung freizulegen. Die Vorrichtung ist so ausgelegt, dass sie eine erste Position einnehmen kann, in der die Nadel für den Transport der Vorrichtung vor der Verwendung geschützt ist. Ferner kann sie eine zweite Position einnehmen, in der die Nadel zum Befüllen der Spritze und zur Injektion freiliegt. Ferner kann sie eine dritte Position einnehmen, in der die Nadel nach dem Befüllen der Spritze, aber vor der Injektion geschützt ist. Ferner kann sie eine vierte Position einnehmen, in der die Nadel nach der Injektion in der Vorrichtung arretiert ist.

### Aufgabe

Der vorliegenden Anmeldung liegt mithin die Aufgabe zugrunde, eine Schutzvorrichtung bereitzustellen, die besonders einfach automatisiert auf eine Nadelträger aufsetzbar ist und zugleich eine hohe Sicherheit gegen ein unbeabsichtigtes Austreten des Nadelrohrs nach dessen Injektionsgebrauch aus der Abdeckung heraus bietet.

### Lösung

Die zugrunde liegende Aufgabe wird erfindungsgemäß mittels eines Verfahrens mit den Merkmalen des Anspruchs 1 gelöst. Vorteilhafte Ausgestaltungen ergeben sich aus den zugehörigen Unteransprüchen.

Die erfindungsgemäße Schutzvorrichtung ist dadurch gekennzeichnet, dass die Halteeinrichtung eine verformbare Lasche, einen der Lasche zugeordneten Anschlag sowie mindestens ein Führungselement umfasst. Die Lasche ist dazu eingerichtet, im Zuge der Überführung der Abdeckung in deren Schutzstellung von dem Nadelrohr verformt und hierdurch in eine erste Richtung verdrängt zu werden, sodass das Nadelrohr in einen hinter der Lasche befindlichen Sicherungsbereich des Innenraums eintreten kann. Das Nadelrohr wird auf diese Weise hinter der Lasche verrastet, sodass die Lasche das Nadelrohr in dem Sicherungsbereich zurückhält und hierdurch eine unbeabsichtigte Freigabe des Nadelrohrs verhindert. Der Anschlag ist gegenüberliegend von der Lasche an der inneren Mantelfläche der Abdeckung bzw. ihrer Wandung ausgebildet und dazu eingerichtet, dass die Lasche im Falle der Verformung durch das Nadelrohr in eine entgegen der ersten Richtung gerichtet zweite Richtung gegen eine untere Anschlagfläche des Anschlags schlägt und hierdurch eine weitere Verformung der Lasche verhindert. Auf diese Weise ist ein unbeabsichtigtes Austreten des Nadelrohrs aus dem Sicherungsbereich verhindert.

In einem unverformten Zustand der Lasche erstreckt sich selbige ausgehend von der inneren Mantelfläche der Abdeckung in den Innenraum, wobei die Lasche - in einem senkrecht zu der Längsachse der Abdeckung geführten Querschnitt betrachtet - mit der Schwenkebene einen Laschenwinkel von mindestens 35° einschließt. Vorzugsweise beträgt dieser Winkel mindestens 40°, weiter vorzugsweise mindestens 45°.

Das mindestens eine Führungselement ist dem Anschlag zugeordnet und dazu eingerichtet, das Nadelrohr, das sich in dem Sicherungsbereich befindet, derart zu führen, dass nach dessen Eintritt in den Sicherungsbereich ein Aufenthalt des Nadelrohrs unterhalb der Anschlagfläche des Anschlags verhindert ist. Insbesondere kann das Führungselement einen Raum unterhalb des Anschlags derart blockieren, dass das Nadelrohr seitlich gegen das Führungselement schlägt und entsprechend nicht in den Raum unterhalb des Anschlags gelangen kann. Auf diese Weise kann verhindert werden, dass das Nadelrohr, nachdem es einmal in den Sicherungsbereich eingetreten ist, sich unter den Anschlag und sodann zwischen die Lasche und der Anschlag bewegt, um auf diese Weise aus dem Sicherungsbereich zu entkommen.

Die erfindungsgemäße Schutzvorrichtung hat viele Vorteile. Insbesondere stellt sie eine sichere Verwahrung des Nadelrohrs innerhalb der Abdeckung sicher, nachdem das Nadelrohr für eine Injektion gebraucht wurde und die Abdeckung daraufhin in ihre Schutzstellung verschwenkt wurde. Dies liegt darin begründet, dass das Nadelrohr nach seinem Eintritt in den Sicherungsbereich, der in der vorstehend beschriebenen Weise das temporäre Verdrängen der elastischen Lasche verlangt, nicht mehr aus dem Sicherungsbereich entkommen kann. Eine Bewegung des Nadelrohrs in Richtung aus dem Sicherungsbereich herausführt unweigerlich dazu, dass das Nadelrohr von einer Unterseite her gegen die Lasche stößt und die Lasche abermals elastisch verformt, diese Verformung jedoch infolge der abgestimmten Geometrie der Lasche und des Anschlags zu einem Anschlag der Lasche gegen die Anschlagfläche des Anschlags führt und hierdurch eine weitere Verformung der Lasche behindert. Mittels des mindestens einen Führungselements ist dabei sichergestellt, dass das Nadelrohr in jedem Fall gegen die Unterseite der Lasche stößt und insbesondere nicht seitlich der Lasche direkt gegen die Anschlagseite des Anschlags stoßen kann. In Versuchen hat sich gezeigt, dass es selbst bei vorsätzlicher Manipulation der Schutzvorrichtung nicht möglich ist, das Nadelrohr nach seinem Eintritt in den Sicherungsbereich wieder aus selbigem zu entfernen.

Ein weiterer Vorteil ist darin zu sehen, dass die Schutzvorrichtung ohne Weiteres auf einen Nadelträger montiert werden kann, der mit einem Nadelrohr ausgestattet ist. Hierbei ist eine Kollision des Nadelrohrs mit der Lasche der Abdeckung, die sich in ihrer Anfangsstellung befindet, ausgeschlossen. Dies wird durch die bezogen auf die Schwenkebene vergleichsweise steile Neigung der Lasche erreicht, die gemäß vorstehender Beschreibung mindestens ° beträgt. Im Stand der Technik sind die bekannten Laschen gegenüber der Schwenkebene deutlich flacher geneigt und erstrecken sich mithin im Wesentlichen mittig durch den Innenraum der Abdeckung. Wenn sich die Abdeckung jedoch in ihrer Anfangsstellung befindet, beansprucht das Nadelrohr diesen Bereich, sodass es im Zuge der Montage einer Abdeckung mit einer solchen flacheren Lasche wahrscheinlich zu einer Kollision des Nadelrohrs mit der Lasche kommen würde.

Ein weiterer Vorteil der erfindungsgemäßen Konstruktion besteht darin, dass die Sicherung des Austritts des Nadelrohrs aus dem Sicherungsbereich in Zusammenwirkung der weichen Lasche mit dem starren Anschlag erfolgt. Dies erlaubt, dass die Lasche als solche vergleichsweise weich, das heißt mit einer geringen Steifigkeit, ausgebildet ist, da die Lasche nicht alleine für die Sicherung des Nadelrohrs verantwortlich ist. Mithin ist eine leichte Verformbarkeit der Lasche für den gewünschten Sicherungseffekt unschädlich, da die Lasche im Zuge ihrer Verformung bei Kontakt mit dem Nadelrohr von der Unterseite der Lasche her zwar leicht verformt werden kann, ein "Durchschlagen" der Lasche in die Gegenrichtung jedoch infolge des Anschlagens der Lasche gegen die Anschlagfläche des Anschlags unterbunden ist. Die leichte Verformbarkeit der Lasche hat gleichwohl den besonderen Vorteil, dass die Schutzvorrichtung auch dann verwendbar ist, wenn ein dünnes Nadelrohr verwendet wird. Insbesondere können Nadelrohre, die beispielsweise für Insulinspritzen verwendet werden, einen besonders kleinen Durchmesser aufweisen, sodass ein solches Nadelrohr besonders leicht verformbar ist. Wäre die Lasche - wie im Stand der Technik üblich - steif ausgebildet, um die Sicherung des Nadelrohrs in dem Sicherungsbereich sicherzustellen, könnte das Nadelrohr die Lasche nicht verdrängen, sondern umgekehrt würde die Lasche das Nadelrohr verformen. Hierdurch wäre ein zuverlässiger Eintritt des Nadelrohrs in den Sicherungsbereich nicht gegeben, wodurch eine mangelnde Sicherheit der jeweiligen Spritze, die mit einer solchen Schutzvorrichtung ausgestattet wäre, gegeben wäre. Diese mangelnde Sicherheit würde nicht darin bestehen, dass das Nadelrohr unbeabsichtigter Weise aus dem Sicherungsbereich austreten kann, sondern darin, dass das Nadelrohr nicht zuverlässig in den Sicherungsbereich eintreten kann. Mittels einer weichen Lasche ist dieses Risiko unterbunden.

In einer vorteilhaften Ausgestaltung der erfindungsgemäßen Schutzvorrichtung liegt - in einem senkrecht zur Längsachse der Abdeckung geführten Querschnitt der Abdeckung betrachtet - zwischen einem von der inneren Mantelfläche der Abdeckung abgewandten Ende der in ihrem unverformten Zustand vorliegenden Lasche und einem der Lasche zugewandten Ende des Anschlags ein in einer zu der Schwenkebene senkrechten Richtung gemessener Abstand vor. Mit anderen Worten liegt bei dieser Ausgestaltung zwischen den einander zugewandten Enden des Anschlags und der Lasche ein senkrecht zu der Schwenkebene gemessener Abstand vor. Dieser Abstand liegt vorzugsweise in einem Bereich zwischen 0,05 mm und 0,25 mm, weiter vorzugsweise zwischen 0,10 mm und 0,20 mm. Somit reicht bei dieser Ausgestaltung die Lasche bei Vorliegen in ihrem unverformten Zustand nicht bis unter die Anschlagfläche des Anschlags. Stattdessen existiert zwischen der Lasche und dem Anschlag ein minimaler Spalt, der einen Eintritt des Nadelrohrs in den Sicherungsbereich des Innenraums der Abdeckung erleichtert. Insbesondere ist eine Verformung der Lasche lediglich von geringem Betrag notwendig, damit das Nadelrohr in den Sicherungsbereich eintreten und hinter der Lasche einschnappen kann. Entsprechend ist diese Ausgestaltung besonders vorteilhaft, um ein Eintreten eines Nadelrohrs in den Sicherungsbereich unter geringer Krafteinwirkung auf das Nadelrohr zu bewerkstelligen, sodass zum einen der Eintritt besonders zuverlässig erfolgen kann und zum anderen eine unbeabsichtigte Verformung des Nadelrohrs vermieden wird.

Ferner kann eine solche Ausgestaltung der Schutzvorrichtung besonders von Vorteil sein, bei der - in einem senkrecht zur Längsachse der Abdeckung geführten Querschnitt der Abdeckung betrachtet - zwischen einem von der inneren Mantelfläche der Abdeckung abgewandten Ende den ihrem unverformten Zustand vorliegenden Lasche und einem der Lasche zugewandten Ende des Anschlags ein in einer zu der Schwenkebene parallelen Richtung gemessener Abstand vorliegt. Dieser Abstand liegt vorzugsweise im Bereich zwischen 0,20 mm und 0,80 mm, weiter vorzugsweise zwischen 0,25 mm und 0,70 mm, noch weiter vorzugsweise zwischen 0,30 mm und 0,60 mm. Diese Ausgestaltung hat ebenfalls den Vorteil, dass der Eintritt eines Nadelrohrs in den Sicherungsbereich vereinfacht ist, da die Lasche lediglich minimal verdrängt werden muss, damit das Nadelrohr hinter der Lasche einschnappt. Grundsätzlich wäre es denkbar, dass das Nadelrohr durch einen infolge des beschriebenen Abstands gebildeten Schlitz zwischen der Lasche und dem Anschlag hindurchtreten und auf diese Weise den Sicherungsbereich verlassen kann; dies ist jedoch mittels des Führungselements verhindert, das das Nadelrohr derart führt, dass es nicht unter den Anschlag gelangen kann und mithin keinen Zugang zu dem gebildeten Schlitz findet. Mithin ist eine Bewegung des Nadelrohrs in eine Richtung aus dem Sicherungsbereich heraus zwingend mit einem Kontakt gegen die Unterseite der Lasche verbunden, die zu der beschriebenen Verformung der Lasche führt, bis diese gegen die Anschlagfläche des Anschlags schlägt.

In bevorzugter Weise erstreckt sich das mindestens eine Führungselement ausgehend von der inneren Mantelfläche der Abdeckung in den Innenraum herein. Dies erfolgt bevorzugt in solcher Weise, dass der Raum unterhalb der Anschlagfläche des Anschlags für das Nadelrohr vollständig blockiert ist. Die sich hierdurch ergebenden Vorteile sind vorstehend bereits dargelegt.

Weiterhin kann es besonders vorteilhaft sein, wenn die Schutzvorrichtung eine Mehrzahl von Führungselementen aufweist, wobei vorzugsweise - in Längsrichtung der Abdeckung betrachtet - mindestens ein Führungselement vor dem Anschlag und mindestens ein Führungselement hinter dem Anschlag angeordnet sind. Diese Ausgestaltung hat den Vorteil, dass eine Bewegung des Nadelrohrs in den Raum unterhalb der Anschlagfläche des Anschlags selbst dann zuverlässig verhindert wird, wenn das Nadelrohr in sich verformt, insbesondere entlang seiner Längsachse verbogen sein sollte. Mithin ist der Raum unter der Anschlagfläche des Anschlags von beiden Seiten des Anschlags her mittels der beiden Führungselemente "versperrt", sodass das Nadelrohr diesen Raum nicht erreichen kann und folglich zuverlässig unterhalb der Lasche gehalten wird.

Die erfindungsgemäße Schutzvorrichtung weiter ausgestaltend ist die Abdeckung in einem senkrecht zu ihrer Längsachse geführten Querschnitt betrachtet mit einer U-förmigen Wandung ausgebildet, die den Innenraum räumlich begrenzt. Hierbei ist die Abdeckung vorzugsweise derart ausgebildet, dass eine Hochachse ihres Querschnitts parallel zu der Schwenkebene der Abdeckung orientiert ist. Insbesondere kann die Abdeckung in ihrem Querschnitt an ihren gegenüberliegenden inneren Mantelflächen, von denen ausgehend sich an der einen Seite die Lasche und an der anderen Seite der Anschlag erstrecken, zumindest im Wesentlichen parallel ausgebildet sein. Eine entsprechende Ausgestaltung ist dem nachstehenden Ausführungsbeispiel entnehmbar. Sie ist besonders von Vorteil, um das Zusammenwirken der Lasche und des Anschlags sicherzustellen.

Weiterhin kann eine solche Ausgestaltung der Schutzvorrichtung besonders vorteilhaft sein, bei der die Basis und die Abdeckung bei Vorliegen der Abdeckung in ihrer Anfangsstellung mittels einer Sollbruchstelle ("Originalitätsverschluss") in Kraft übertragender Weise miteinander verbunden sind. Die Sollbruchstelle ist unter Aufbringung einer händischen Kraft zerstörbar, sodass die Abdeckung nach der Zerstörung der Sollbruchstelle bestimmungsgemäß in ihre Nutzstellung verschwenkbar ist. Diese Ausgestaltung ist besonders von Vorteil, um das Nadelrohr vor dessen Injektionsgebrauch sicher innerhalb der Abdeckung zu lagern, wobei mittels der Sollbruchstelle ein versehentliches Verschwenken der Abdeckung in die Nutzstellung und mithin eine versehentliche Freigabe des Nadelrohrs unterbunden ist. Ferner ist es für den Schutz des Nadelrohrs vor dessen Injektionsgebrauch nicht erforderlich, eine separate Abdeckung zu verwenden, die beispielsweise in Form einer geschlossenen Kappe über das Nadelrohr gestülpt wird. Stattdessen kann die Funktion des Schutzes des Nadelrohrs sowohl vor dem Injektionsgebrauch als auch nach dem Injektionsgebrauch allein von der Abdeckung übernommen werden. Dies ist aufgrund der vorstehend erläuterten Ausgestaltung der steil gegenüber der Schwenkebene geneigten Lasche besonders gut möglich, da die beschriebene Gefahr einer Kollision des Nadelrohrs mit der Lasche im Zuge einer Montage der Schutzvorrichtung auf einen Nadelträger ausgeräumt ist. Wenn die Sollbruchstelle zerstört ist, dient sie im Übrigen als Anzeiger dafür, dass das Nadelrohr womöglich bereits benutzt wurde. Ein Benutzer der Schutzvorrichtung würde daher ein Nadelrohr nicht mehr benutzen, wenn er oder sie nicht selbst die Schutzvorrichtung "geöffnet" hat, das heißt die Sollbruchstelle zerstört hat.

In einer weiterhin vorteilhaften Ausgestaltung der erfindungsgemäßen Schutzvorrichtung sind die Abdeckung und die Basis unter Ausbildung eines Folienscharniers miteinander verbunden. Ein solches Folienscharnier ist besonders gut geeignet, die beschriebene Verschwenkbarkeit der Abdeckung relativ zu der Basis herzustellen, wobei gleichzeitig eine einstückige Ausgestaltung der Schutzvorrichtung erreicht werden kann. Mithin ist es besonders von Vorteil, wenn die Schutzvorrichtung einstückig ausgebildet ist.

Weiterhin kann eine solche Ausgestaltung der Schutzvorrichtung besonders vorteilhaft sein, bei der die Abdeckung und die Basis bei Vorliegen der Abdeckung in ihrer Anfangsstellung koaxial zueinander angeordnet sind. Bei dieser Ausgestaltung sind folglich Längsachsen der Basis und der Abdeckung parallel zueinander orientiert. Vorzugsweise fallen die Längsachsen der Abdeckung und der Basis zusammen.

Alternativ oder zusätzlich hierzu kann es besonders vorteilhaft sein, wenn die Abdeckung bei Vorliegen in ihrer Schutzstellung derart in die zweite Schwenkrichtung verschwenkt ist, dass die Längsachsen der Basis und der Abdeckung nicht-parallel zueinander orientiert sind, das heißt einen Winkel >0° einschließen. Diese Ausgestaltung hat den Vorteil, dass das Nadelrohr besonders einfach mit der Lasche in Kontakt gebracht werden kann, wodurch die Lasche in beschriebener Weise verdrängt wird, sodass das Nadelrohr schließlich in den Sicherungsbereich des Innenraums der Abdeckung eintritt. Bei einer Ausgestaltung, bei der die Abdeckung im Vergleich zwischen ihrer Anfangsstellung und ihrer Schutzstellung unterschiedlich gegenüber der Basis geneigt ist, ist es besonders einfach möglich, zunächst bei der Montage der Schutzvorrichtung an einem Nadelträger eine Kollision des Nadelrohrs mit der Lasche zu vermeiden, eine solche Kollision jedoch bei der Überführung der Abdeckung in die Schutzstellung bewusst zu provozieren, um das Nadelrohr in den Sicherungsbereich eintreten zu lassen. Weiterhin ist die beschriebene Ausführung insoweit vorteilhaft, als die Abdeckung zwecks Arretierung in ihrer Schutzstellung besonders einfach an der Basis befestigt werden kann, bevorzugt unter Ausbildung eines Formschlusses.

Entsprechend kann es besonders von Vorteil sein, wenn an der Basis mindestens eine Rastmittel ausgebildet ist, das dazu eingerichtet ist, formschlüssig mit mindestens einem Rastpartner der Abdeckung zusammenzuwirken, sodass die Abdeckung in der Schutzstellung arretierbar ist. Das Zusammenwirken des Rastmittels mit dem Rastpartner ist besonders einfach realisierbar, indem die Abdeckung bei ihrer Überführung in die Schutzstellung über die Anfangsstellung hinaus relativ zu der Basis verschwenkt und hierdurch der Rastpartner der Abdeckung mit dem Rastmittel der Basis in Kontakt gebracht wird. Infolge des Eingriffs des Rastpartners mit dem Rastmittel ist sichergestellt, dass die Abdeckung nicht in unbeabsichtigter Weise ausgehend von ihrer Schutzstellung zurück in ihre Nutzstellung überführt wird.

Die zugrunde liegende Aufgabe wird ferner erfindungsgemäß mittels eines Sets mit den Merkmalen des Anspruchs 12 gelöst. Eine vorteilhafte Ausgestaltung ergibt sich aus dem zugehörigen Unteranspruch.

Das Set umfasst eine erfindungsgemäße Schutzvorrichtung gemäß der vorstehenden Beschreibung sowie einen Nadelträger mit einem darin gelagerten Nadelrohr. Die Basis der Schutzvorrichtung ist formschlüssig mit dem Nadelträger verbunden, sodass das Nadelrohr bei Vorliegen der Abdeckung in ihrer Anfangsstellung parallel zu der Längsachse der Abdeckung orientiert ist. Mittels Verschwenkens der Abdeckung von ihrer Anfangsstellung in ihre Nutzstellung in eine erste Schwenkrichtung in der Schwenkebene kann das Nadelrohr zum Injektionsgebrauch freigegeben werden. Im Anschluss daran kann die Abdeckung in eine der ersten Schwenkrichtung entgegengesetzte zweite Schwenkrichtung zurück in Richtung der Anfangsstellung verschwenkt werden, sodass das Nadelrohr wieder in den Innenraum der Abdeckung eintritt und vor einem unbeabsichtigten Kontakt mit einer Person geschützt ist. Wie vorstehend dargelegt, kann es hierbei besonders vorteilhaft sein, wenn die Abdeckung bei Erreichen ihrer Schutzstellung relativ zu der Basis arretiert wird, sodass eine abermalige Überführung in die Nutzstellung verhindert ist. Die Überführung der Abdeckung in die Schutzstellung geht mit einem Eintritt des Nadelrohrs in den Sicherungsbereich jenseits der Lasche einher, wobei die Lasche infolge eines Kontakts mit dem Nadelrohr elastisch verformt und hierdurch verdrängt wird, bis das Nadelrohr hinter der Lasche einschnappt und sich daraufhin in dem Sicherungsbereich befindet.

In einer besonders vorteilhaften Ausgestaltung des erfindungsgemäßen Sets befindet sich eine Laschenbasis der Lasche - in einem senkrecht zu der Längsachse der Abdeckung geführten Querschnitt der Abdeckung betrachtet - zumindest bei Vorliegen der Abdeckung in ihrer Anfangsstellung mit dem Nadelrohr in einer gemeinsamen, senkrecht zu der Schwenkebene orientierten Ebene. Die Laschenbasis bezeichnet hierbei einen proximalen Teil der Lasche, mit der die Lasche an die innere Mantelfläche der Wandung der Abdeckung angeschlossen ist. Die beschriebene Ausgestaltung hat den Vorteil, dass eine Kollision der Lasche mit dem Nadelrohr im Zuge der Montage der Schutzvorrichtung an dem Nadelträger ausgeschlossen ist, da sich das Nadelrohr und die Laschenbasis bezogen auf die Schwenkebene der Abdeckung auf einen gemeinsamen "Höhenniveau" befinden. Da sich die Lasche jedoch geneigt bezogen auf die Schwenkebene erstreckt und sich das Nadelrohr typischerweise mittig durch den Innenraum der Abdeckung erstreckt, erstreckt sich die Lasche gezwungenermaßen an dem Nadelrohr vorbei in Richtung der Schwenkebene. Eine entsprechende Ausgestaltung ist nachstehend anhand des Ausführungsbeispiels veranschaulicht.

### Ausführungsbeispiele

Die Erfindung ist nachstehend anhand eines Ausführungsbeispiels, das in den Figuren dargestellt ist, näher erläutert. Es zeigt:
- Fig. 1:: Eine Ansicht einer erfindungsgemäßen Schutzvorrichtung, wobei sich eine Abdeckung in einer Anfangsstellung befindet,
- Fig. 2:: Ein perspektivischer Querschnitt durch die Schutzvorrichtung gemäß Figur 1,
- Fig. 3:: Eine perspektivische Ansicht eines Teils der Schutzvorrichtung gemäß Figur 1,
- Fig. 4:: Ein Querschnitt durch die Schutzvorrichtung gemäß Figur 1,
- Fig. 5:: Eine Ansicht der Schutzvorrichtung gemäß Figur 1, wobei sich die Abdeckung in einer Nutzstellung befindet,
- Fig. 6:: Eine perspektivische Ansicht der Schutzvorrichtung gemäß Figur 5,
- Fig. 7:: Eine perspektivische Ansicht eines Teils der Schutzvorrichtung gemäß Figur 1, wobei sich die Abdeckung in einer Schutzstellung befindet,
- Fig. 8:: Ein Querschnitt durch die Abdeckung der Schutzvorrichtung gemäß Figur 7.

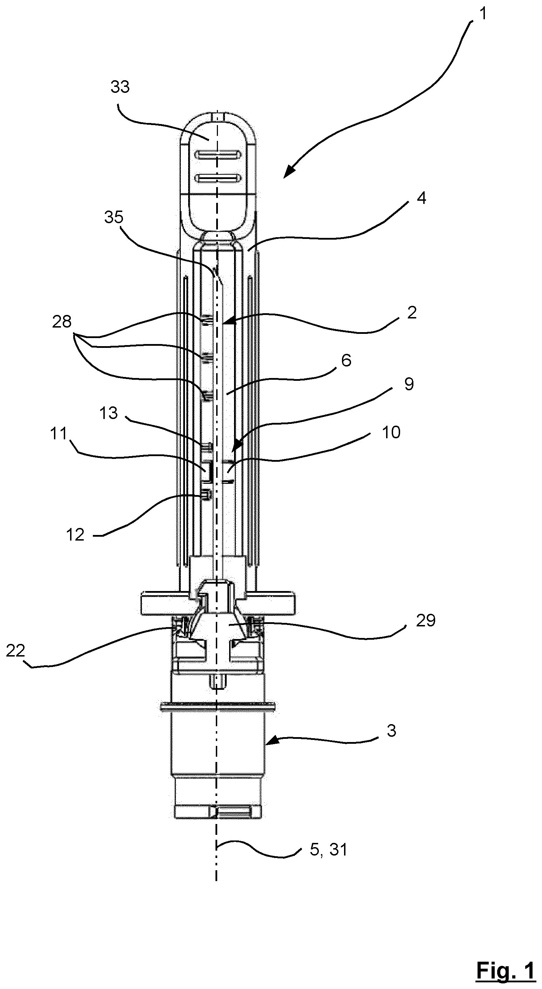

Ein Ausführungsbeispiel, das in den **Figuren 1 bis 8** gezeigt ist, umfasst eine erfindungsgemäße Schutzvorrichtung **1,** die eine Basis **3** sowie eine Abdeckung **4** umfasst. Die Abdeckung **4** ist relativ zu der Basis **3** verschwenkbar ausgebildet, wobei die Abdeckung **4** mittels eines Folienscharniers **22** an die Basis 3 angeschlossen ist. Die Schutzvorrichtung **1** ist einstückig ausgebildet und wurde als Spritzgussteil hergestellt.

Die Basis **3** ist dazu geeignet, mit einem Nadelträger **25** zusammenzuwirken, mittels dessen ein Nadelrohr **2** getragen ist. Insbesondere ist das Nadelrohr **2** in eine komplementäre Ausnehmung des Nadelträgers **25** eingeklebt, sodass sich eine Längsachse des langgestreckt ausgebildet Nadelrohrs **2** parallel zu einer Längsachse **31** der Basis **3** erstreckt. Dies ergibt sich besonders gut anhand von **Figur 1****.**

Die Schutzvorrichtung **1** wird als Ganzes an dem Nadelträger **25** montiert, wobei diese Montage bevorzugt automatisiert erfolgt. Die Schutzvorrichtung **1** befindet sich bei der Montage in einem Ausgangszustand, in dem sich die Abdeckung **4** in einer Anfangsstellung befindet. In dieser Anfangsstellung ist die Abdeckung **4** mittels einer Sollbruchstelle **21** an der Basis **3** befestigt, sodass eine Verschwenkung der Abdeckung **4** relativ zu der Basis **3** blockiert ist. Bei Vorliegen der Abdeckung **4** in ihrer Anfangsstellung sind eine Längsachse **5** der Abdeckung **4** und die Längsachse **31** der Basis **3** parallel zueinander orientiert. In dem gezeigten Beispiel ist die Abdeckung **4** ferner koaxial zu der Basis **3** ausgebildet, sodass die Längsachsen **5, 31** der Abdeckung **4** und der Basis **3** zusammenfallen.

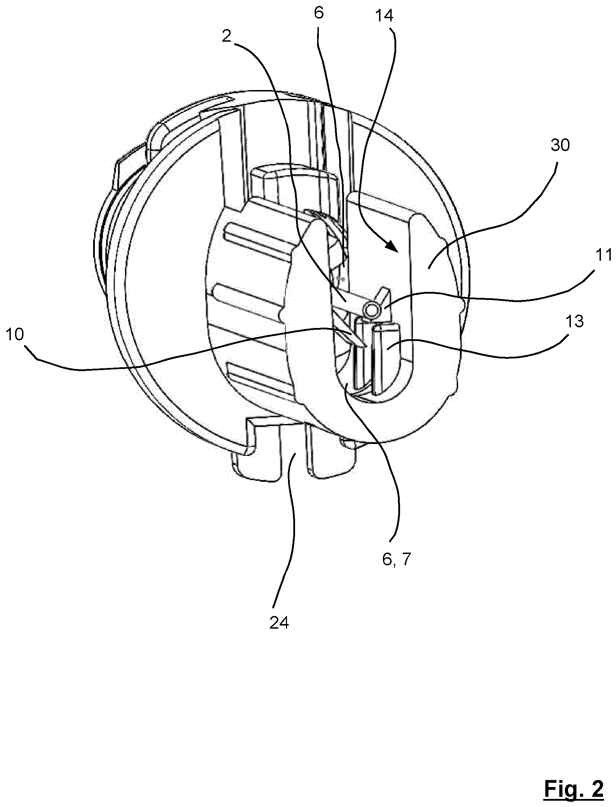

Nach der Montage der Schutzvorrichtung **1** auf dem Nadelträger **25** befindet sich das Nadelrohr **2** innerhalb eines Innenraums **6** der Abdeckung **4,** der von einer Wandung **30** der Abdeckung **4** eingefasst ist. Dies ergibt sich besonders gut anhand von **Figur 2****.** Die Lagerung des Nadelrohrs **2** innerhalb der Abdeckung **4** hat den Vorteil, dass eine Person, die mit einer Spritze umgeht, an der der Nadelträger **25** angebracht ist, vor einer Verletzung mit bzw. an einer Nadelspitze **35** des Nadelrohrs **2** geschützt ist. Aufgrund der Blockade der Schwenkbewegung der Abdeckung **4** relativ zu der Basis **3** mittels der Sollbruchstelle **21** ist zudem sichergestellt, dass eine unbeabsichtigte Freigabe des Nadelrohrs **2** verhindert ist.

Um das Nadelrohr **2** einem Injektionsgebrauch zuzuführen, ist es erforderlich, das Nadelrohr **2** schließlich freizugeben. Hierzu kann der Benutzer der jeweiligen Spritze die Abdeckung **4** unter Aufbringung einer händischen Kraft an einer Grifflasche **33** der Abdeckung **4** von der Basis **3** "freibrechen", wobei die Sollbruchstelle **21** zerstört wird. Hierdurch ist die Verschwenkbarkeit der Abdeckung **4** unter Verwendung des Folienscharniers **22** relativ zu der Basis **3** freigegeben. Entsprechend kann die Abdeckung **4** nunmehr mittels einer Verschwenkung in eine erste Schwenkrichtung innerhalb der Schwenkebene **8** in ihre Nutzstellung überführt werden, in der das Nadelrohr **2** freigegeben ist. Die Abdeckung **4** ist hierbei innerhalb der Schwenkebene **8** relativ zu der Basis **3** verschwenkbar, wobei eine Wandung **30** der Abdeckung **4** in der Schwenkebene **8** an einer Seite offen ist, sodass das Nadelrohr **2** im Zuge der Verschwenkung der Abdeckung **4** ohne Weiteres aus dem Innenraum **6** austreten kann. Dies ergibt sich besonders gut anhand von **Figur 2****.** Die Wandung **30** ist in dem gezeigten Beispiel U-förmig ausgebildet, wobei eine Hochachse **20** eines Querschnitts der Wandung **30** parallel zu der Schwenkebene **8** sowie senkrecht zu der Längsachse **5** der Abdeckung **4** orientiert ist.

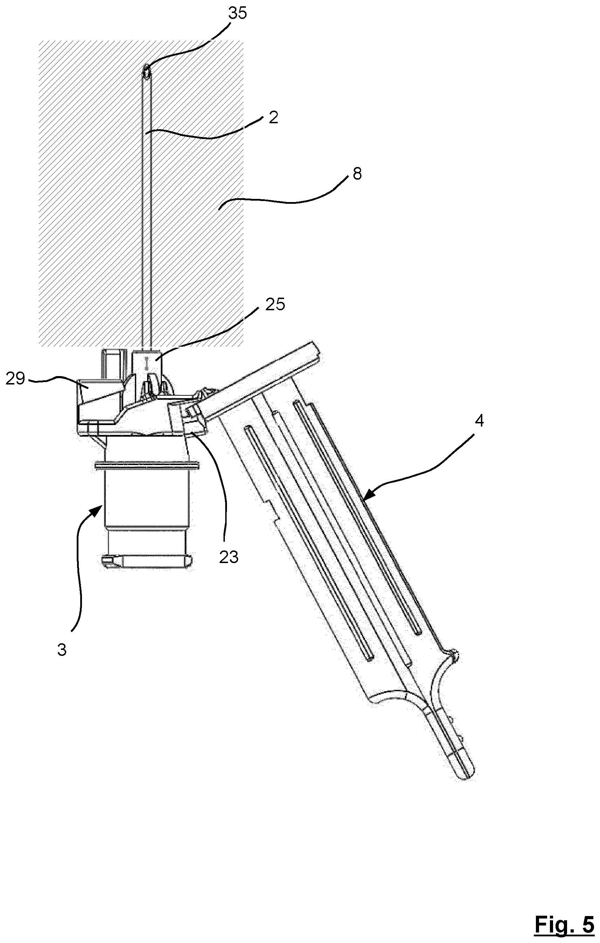

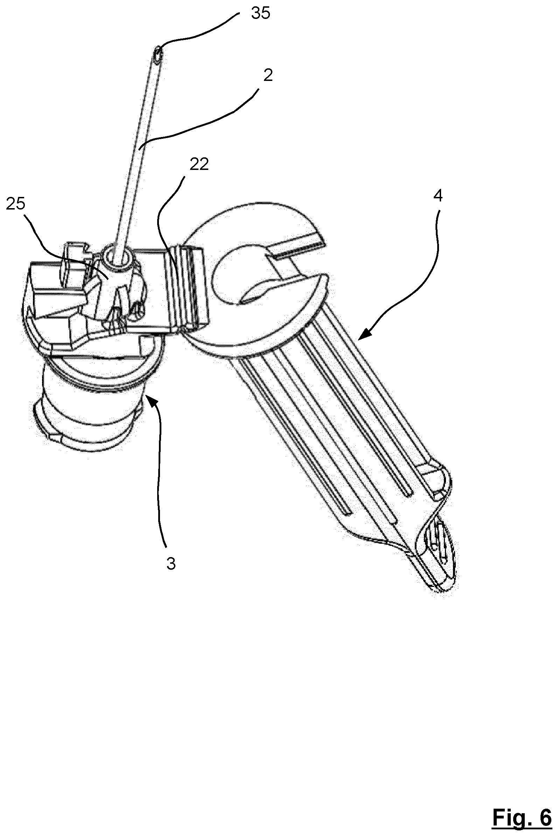

Bei Vorliegen der Abdeckung **4** in ihre Nutzstellung, die sich besonders gut anhand der Figuren **5** **und** **6** ergibt, ist das Nadelrohr **2** gemäß vorstehender Erläuterung freigegeben, sodass es für seine Injektionsgebrauch vorbereitet ist. In dem gezeigten Beispiel ist die Basis **3** mit einem Rastmittel **23** ausgestattet, das dazu eingerichtet ist, mit einem Rastpartner **24** zusammenzuwirken, der an der Abdeckung **4** ausgebildet ist. Das Rastmittel **23** und der Rastpartner **24** können auf diese Weise eine formschlüssige Verbindung miteinander eingehen, wodurch die Abdeckung **4** bei Vorliegen in ihrer Nutzstellung an der Basis **3** arretierbar ist. Auf diese Weise ist verhindert, dass während eines Injektionsgebrauchs des Nadelrohrs **2** die Abdeckung **4** unkontrolliert bewegt wird und hierdurch den Benutzer der jeweiligen Spritze stört.

Nach dem Injektionsgebrauch des Nadelrohrs **2** wird die Abdeckung **4** ausgehend von ihrer Nutzstellung in eine der ersten Schwenkrichtung entgegengesetzte zweite Schwenkrichtung zurück relativ zu der Basis **3** verschwenkt, bis die Abdeckung **4** ihre Schutzstellung einnimmt. Für die Schutzstellung der Abdeckung **4** ist wesentlich, dass die Abdeckung **4** so weit in die zweite Schwenkrichtung zurück verschwenkt wird, dass die das Nadelrohr **2** wieder in den Innenraum **6** der Abdeckung **4** eintritt und hierdurch vor einem unbeabsichtigten Kontakt mit einer Person geschützt ist. Da das Nadelrohr **2** zwischenzeitlich mit Blut einer jeweiligen Person, der mittels der Spritze ein Medikament injiziert wurde, in Kontakt gekommen sein kann, ist der Schutz vor einem sich daran anschließenden Kontakt mit dem Nadelrohr **2** von wesentlicher Bedeutung. Entsprechend ist es notwendig, dass die Abdeckung **4** das Nadelrohr **2** nach Überführung in die Schutzstellung derart aufnimmt, dass eine unbeabsichtigte Freigabe des Nadelrohrs **2** im Anschluss daran unmöglich ist.

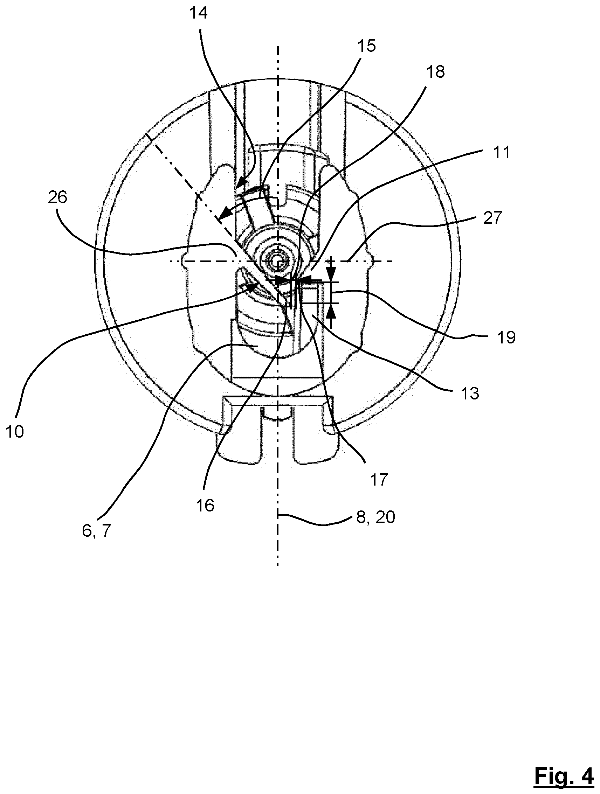

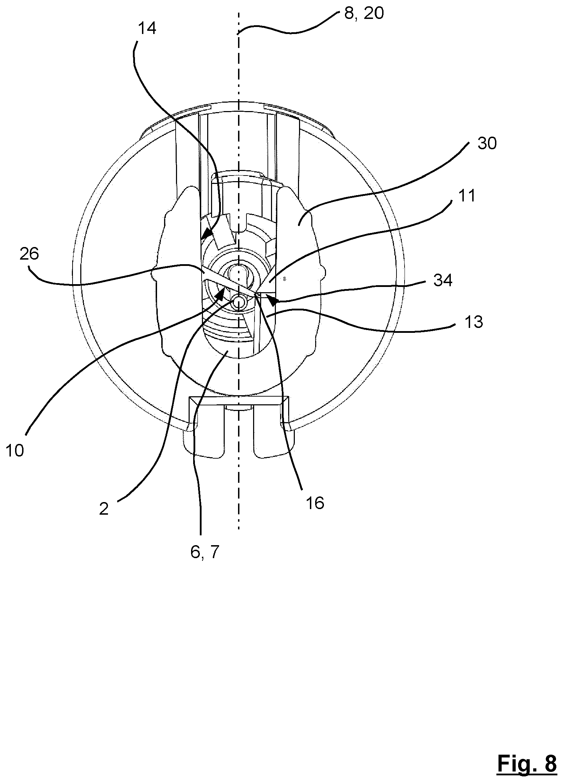

Um dies zu gewährleisten, umfasst die Abdeckung **4** eine Halteeinrichtung **9,** die eine Lasche **10,** einen Anschlag **11** sowie zwei Führungselemente **12, 13** umfasst. Die Halteeinrichtung **9** ist besonders gut anhand der Querschnitte gemäß den **Figuren 4** **und** **8** erkennbar. Zunächst bei Vorliegen der Abdeckung **4** in ihrer Anfangsstellung befindet sich das Nadelrohr **2** in einem Bereich des Innenraums **6,** der diesseits der Lasche **10** und mithin außerhalb eines Sicherungsbereichs **7** des Innenraums **6** ausgebildet ist. In diesem Zustand ist ein Austritt des Nadelrohrs **2** aus dem Innenraum **6** nicht verhindert, da das Nadelrohr **2** noch nicht für eine Injektion verwendet wurde. Gemäß vorstehender Erläuterung soll entsprechend die Abdeckung **4** dazu geeignet sein, in eine Nutzstellung überführt zu werden und hierbei das Nadelrohr **2** bestimmungsgemäß freizugeben.

Im Zuge der Verschwenkung der Abdeckung **4** ausgehend von der Nutzstellung zurück in die Schutzstellung wird hingegen mittels der Halteeinrichtung **9** bewirkt, dass das Nadelrohr **2** sicher in dem Innenraum **6** der Abdeckung **4** gehalten wird. Hierzu wird in dem gezeigten Beispiel die Abdeckung **4** über die Anfangsstellung hinaus in die zweite Schwenkrichtung verschwenkt, sodass die Längsachsen **5, 31** der Abdeckung **4** sowie der Basis 3 gemeinsam einen Winkel **32** einschließen und folglich nicht parallel zueinander orientiert sind. Der Winkel **32** beträgt in dem gezeigten Beispiel ca. 20°. Diese Art der Bewegung der Abdeckung **4** führt dazu, dass das Nadelrohr **2** nicht nur wieder an der offenen Seite der Wandung **30** der Abdeckung **4** in den Innenraum **6** eintritt, sondern zudem in den Sicherungsbereich **7** jenseits der Lasche **10** eintaucht. Dies erfolgt derart, dass die Lasche **10** bei ihrem erstmaligen Kontakt mit dem Nadelrohr **2** verdrängt wird, wobei sich die Lasche **10** aufgrund ihrer elastischen Eigenschaften verformt. Ab einer bestimmten Stellung der Abdeckung **4** schnappt das Nadelrohr **2** schließlich hinter der Lasche **10** ein, die sich daraufhin elastisch zurück in ihre ursprüngliche Form verformt. Dieser Zustand ist besonders gut anhand von **Figur 8** erkennbar.

Eine Bewegung des Nadelrohrs **2** aus dem Sicherungsbereich **7** heraus ist daraufhin nicht mehr möglich. Dies liegt darin begründet, dass das Nadelrohr **2** selbst bei einer erzwungenen Bewegung der Abdeckung **4** in die erste Schwenkrichtung, das heißt in Richtung der Nutzstellung, gegen eine Unterseite der Lasche **10** schlägt und diese entsprechend elastisch in Richtung der offenen Seite der Wandung **30** verformt. Im Zuge dieser Verformung schlägt die Lasche **10** zumindest mit einem der Wandung **30** abgewandten Ende **16** gegen eine untere Anschlagfläche **34** des Anschlags **11,** wodurch eine weitergehende Verformung der Lasche **10** verhindert ist. Diese Art der Zusammenwirkung von Lasche **10** und Anschlag **11** ist in dem gezeigten Beispiel dadurch realisiert, dass sich die Lasche **10** und der Anschlag **11** ausgehend von einander gegenüberliegenden inneren Mantelflächen **14** der Wandung **30** der Abdeckung **4** erstrecken. Die Führungselemente **12, 13,** die hier entlang der Längsachse **5** der Abdeckung **4** betrachtet beidseits des Anschlags **11** angeordnet sind sowie dem Anschlag **11** zugeordnet sind, verhindern, dass das Nadelrohr **2** nach seinem Eintritt in den Sicherungsbereich **7** in einen Raum unterhalb des Anschlags **11** gelangen kann. Mithin ist ein unmittelbarer Kontakt des Nadelrohrs **2** mit der Anschlagfläche **34** des Anschlags **11** nicht möglich. Hierdurch ist verhindert, dass das Nadelrohr **2** sich von einer Seite der Lasche **10** her bis oberhalb des Endes **16** der Lasche **10** bewegen und hierdurch aus dem Sicherungsbereich **7** entkommen kann.

Erfindungsgemäß erstreckt sich die Lasche **10** bezogen auf die Schwenkebene **8** unter einem Laschenwinkel **15,** der in dem gezeigten Beispiel 40° beträgt. Ferner ist die Lasche **10** bezogen auf das Nadelrohr **2** derart an der Wandung **30** angeordnet, dass eine Laschenbasis **26** sich bei Vorliegen der Abdeckung **4** in deren Anfangsstellung zumindest im Wesentlichen auf demselben Höhenniveau wie das Nadelrohr **2** befindet. Dies äußert sich dadurch, dass die Laschenbasis **26** und das Nadelrohr **2** bei Vorliegen der Abdeckung **4** in ihrer Anfangsstellung sich innerhalb einer gemeinsamen Ebene **27** befinden, die senkrecht zu der Schwenkebene **8** orientiert ist. Die beschriebene Ausgestaltung führt dazu, dass im Zuge einer Montage der Schutzvorrichtung **1** an einem Nadelträger **25** eine Kollision des Nadelrohrs **2** mit der Lasche **10** ausgeschlossen ist. Dies ergibt sich besonders gut anhand von **Figur 4****,** in der deutlich ein entsprechender Abstand zwischen dem Nadelrohr **2** und der Lasche **10** erkennbar ist.

Um den Eintritt des Nadelrohrs **2** in den Sicherungsbereich **7** weiter zu vereinfachen, ist es in der vorliegenden Ausführungsform vorgesehen, dass zwischen dem Ende **16** der Lasche **10,** das der Laschenbasis **26** abgewandt ist, und einem Ende **17** des Anschlags **11,** das der Lasche **10** zugewandt ist, ein senkrecht zu der Schwenkebene **8** gemessener Abstand **18** besteht. Mit anderen Worten liegt zwischen der Lasche **10** und dem Anschlag **11** in Richtung senkrecht zu der Schwenkebene **8** betrachtet eine "Lücke" vor. Dasselbe gilt für die Richtung parallel zu der Schwenkebene **8,** in der betrachtet zwischen den beiden Enden **16, 17** der Lasche **10** sowie des Anschlags **11** ein Abstand **19** vorliegt. Der senkrecht zu der Schwenkebene **8** gemessene Abstand **18** beträgt in dem gezeigten Beispiel ca. 0,15 mm, während der Abstand **19** ca. 0,40 mm beträgt. Diese Ausgestaltung hat sich als besonders zweckmäßig herausgestellt, um das beschriebene Kollisionsrisiko zwischen der Lasche **10** und dem Nadelrohr **2** zu minimieren, einen leichten Übertritt des Nadelrohrs **2** in den Sicherungsbereich **7** zu ermöglichen und ferner einen sicheren Anschlag der Lasche **10** gegen die Anschlagfläche **34** des Anschlags **11** zu bewirken. Betreffend das Eintreten des Nadelrohrs **2** in den Sicherungsbereich **7** hat sich insbesondere gezeigt, dass ein Kraftaufwand, den das Nadelrohr **2** auf die Lasche **10** aufbringen muss, um letztere in solcher Weise zu verformen, dass der Eintritt in den Sicherungsbereich **7** erfolgen kann, besonders gering ist. Dies hat den Vorteil, dass die Schutzvorrichtung **1** auch für die Injektion solcher Mittel geeignet ist, die mit Nadelrohren **2** mit äußerst geringem Durchmesser injiziert werden. Dies gilt beispielsweise für Insulinspritzen, für die Nadelrohr mit einem Durchmesser von weniger als 0,2 mm verwendet werden.

In besonders bevorzugter Weise umfasst die gezeigte Schutzvorrichtung **1** neben den Führungselementen **12, 13** weitere Führungselemente **28,** die entlang der Längsachse **5** der Abdeckung **4** verteilt in dem Innenraum **6** angeordnet sind. Diese Führungselemente **28** erstrecken sich analog zu den Führungselementen **12, 13** ausgehend von der inneren Mantelfläche **14** der Wandung **30** der Abdeckung **4.** Dies ergibt sich besonders gut anhand von **Figur 1****.**

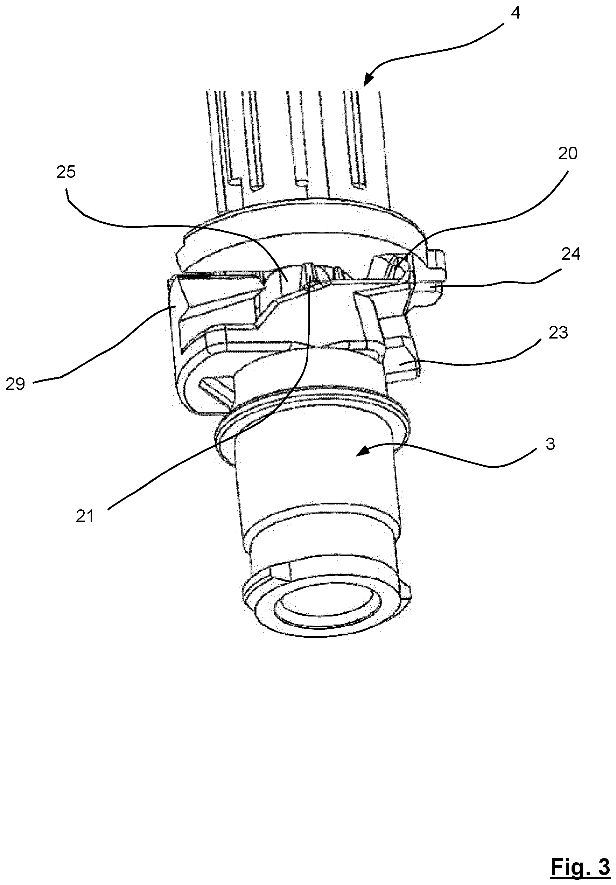

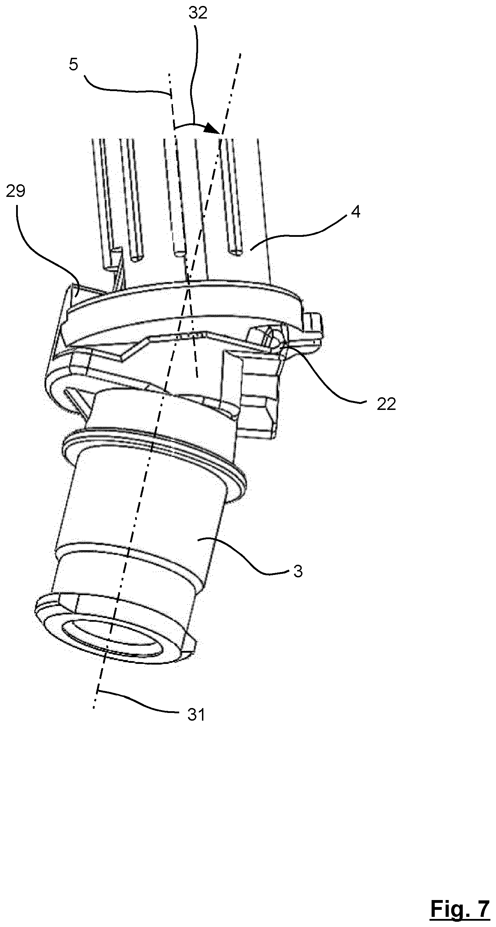

Weiterhin verfügt in dem gezeigten Beispiel die Basis **3** über ein Sicherungselement **29,** das sich besonders gut anhand der **Figuren 3** **und** **7** ergibt. Das Sicherungselement **29** dient dazu, die Abdeckung **4** im Zuge ihrer Überführung in die Schutzstellung formschlüssig an der Basis **2** verrasten, sodass eine unbeabsichtigte Verschwenkung der Abdeckung **4** in die erste Schwenkrichtung zurück in eine Nutzstellung verhindert ist. Hierfür würde es einer vorsätzlichen Kraftaufwendung auf die Abdeckung **4** bedürfen, um den Formschluss mit dem Sicherungselement **29** aufzulösen. In diesem Fall würde gleichwohl das Nadelrohr **2** nicht freigegeben werden, da es mittels der Halteeinrichtung **9** zuverlässig in dem Sicherungsbereich **7** des Innenraum **6** gehalten ist. In Versuchen hat sich gezeigt, dass sich das Nadelrohr **2** in diesem Fall verbiegt.

### Bezugszeichenliste

- 1: Schutzvorrichtung
- 2: Nadelrohr
- 3: Basis
- 4: Abdeckung
- 5: Längsachse
- 6: Innenraum
- 7: Sicherungsbereich
- 8: Schwenkebene
- 9: Halteeinrichtung
- 10: Lasche
- 11: Anschlag
- 12: Führungselement
- 13: Führungselement
- 14: Mantelfläche
- 15: Laschenwinkel
- 16: Ende
- 17: Ende
- 18: Abstand
- 19: Abstand
- 20: Hochachse
- 21: Sollbruchstelle
- 22: Folienscharnier
- 23: Rastmittel
- 24: Rastpartner
- 25: Nadelträger
- 26: Laschenbasis
- 27: Ebene
- 28: Führungselement
- 29: Sicherungselement
- 30: Wandung
- 31: Längsachse
- 32: Winkel
- 33: Grifflasche
- 34: Anschlagfläche
- 35: Nadelspitze

## Patentansprüche

1. Schutzvorrichtung (1) für ein Nadelrohr (2) einer Spritze, umfassend
- eine Basis (3) und
- eine mit der Basis (3) verbundene Abdeckung (4),
wobei die Basis (3) dazu eingerichtet ist, mit einem Nadelträger (25) formschlüssig zusammenzuwirken, in dem das Nadelrohr (2) gelagert ist,
wobei die Abdeckung (4) entlang einer Längsachse (5) langgestreckt ausgebildet ist und einen Innenraum (6) begrenzt, in dem das Nadelrohr (2) zum Schutz einer Person vor einem unbeabsichtigten Kontakt mit dem Nadelrohr (2) lagerbar ist,
wobei die Abdeckung (4) schwenkbar an der Basis (3) gelagert ist, sodass die Abdeckung (4) innerhalb einer Schwenkebene (8) ausgehend von einer Anfangsstellung, in der das Nadelrohr (2) vor einem Injektionsgebrauch in dem Innenraum (6) gelagert ist, relativ zu der Basis (3) in eine erste Schwenkrichtung in eine Nutzstellung, in der das Nadelrohr (2) zum Injektionsgebrauch freigegeben ist, und schließlich in eine der ersten Schwenkrichtung entgegen gesetzte zweite Schwenkrichtung in eine Schutzstellung überführbar ist, in der das Nadelrohr (2) nach dem Injektionsgebrauch wieder in dem Innenraum (6) gelagert ist,
wobei die Abdeckung (4) in der Schwenkebene (8) an einer Seite offen ist, sodass das Nadelrohr (2) infolge der Schwenkbewegungen der Abdeckung (4) zunächst zum Injektionsgebrauch aus dem Innenraum (6) austreten und nach dem Injektionsgebrauch wieder in den Innenraum (6) eintreten kann,
wobei die Abdeckung (4) in dem Innenraum (6) eine Halteeinrichtung (9) aufweist, mittels der das Nadelrohr (2) im Zuge der Überführung der Abdeckung (4) in die Schutzstellung derart formschlüssig verrastbar ist, dass nach erfolgter Überführung der Abdeckung (4) in die Schutzstellung ein unbeabsichtigtes Austreten des Nadelrohrs (2) aus dem Innenraum (6) unterbunden ist,
wobei die Halteeinrichtung (9) eine verformbare Lasche (10), einen der Lasche (10) zugeordneten Anschlag (11) sowie mindestens ein Führungselement (12, 13) aufweist,
wobei die Lasche (10) dazu eingerichtet ist, im Zuge der Überführung der Abdeckung (4) in deren Schutzstellung von dem Nadelrohr (2) verformt und hierdurch in eine erste Richtung verdrängt zu werden, sodass das Nadelrohr (2) in einen hinter der Lasche (10) befindlichen Sicherungsbereich (7) des Innenraums (6) eintreten kann, in dem das Nadelrohr (2) hinter der Lasche (10) verrastet ist,
wobei der Anschlag (11) gegenüberliegend von der Lasche (10) an der inneren Mantelfläche (14) der Abdeckung (4) ausgebildet und dazu eingerichtet ist, dass die Lasche (10) im Falle der Verformung durch das Nadelrohr (2) in eine entgegen der ersten Richtung gerichtete zweiten Richtung gegen eine Anschlagfläche (34) des Anschlags (11) schlägt, wodurch ein unbeabsichtigtes Austreten des Nadelrohrs (2) aus dem Sicherungsbereich (7) verhindert ist,
wobei das Führungselement (12, 13) dem Anschlag (11) zugeordnet und dazu eingerichtet ist, das Nadelrohr (2) derart zu führen, dass nach dessen Eintritt in den Sicherungsbereich (7) ein Aufenthalt des Nadelrohrs (2) unterhalb der Anschlagfläche (34) des Anschlags (11) verhindert ist,
**dadurch gekennzeichnet, dass**
sich die Lasche (10) bei Vorliegen in einem unverformten Zustand ausgehend von einer inneren Mantelfläche (14) der Abdeckung (4) in den Innenraum (6) erstreckt und - in einem senkrecht zu der Längsachse (5) der Abdeckung (4) geführten Querschnitt betrachtet - mit der Schwenkebene (8) einen Laschenwinkel (15) von mindestens 35° einschließt.

2. Schutzvorrichtung (1) nach Anspruch 1, **dadurch gekennzeichnet, dass** der Laschenwinkel (15) mindestens 37,5°, vorzugsweise mindestens 40°, beträgt.

3. Schutzvorrichtung (1) nach einem der vorhergehenden Ansprüche, **dadurch gekennzeichnet, dass** - in einem senkrecht zu der Längsachse (5) der Abdeckung (4) geführten Querschnitt der Abdeckung (4) betrachtet - zwischen einem von der inneren Mantelfläche (14) der Abdeckung (4) abgewandten Ende (16) der in ihrem unverformten Zustand vorliegenden Lasche (10) und einem der Lasche (10) zugewandten Ende (17) des Anschlags (11) ein in einer zu der Schwenkebene (8) senkrechten Richtung gemessener Abstand (18) vorliegt, wobei vorzugsweise der Abstand (18) im Bereich zwischen 0,05 mm und 0,25 mm, vorzugsweise zwischen 0,10 mm und 0,20 mm, beträgt.

4. Schutzvorrichtung (1) nach einem der vorhergehenden Ansprüche, **dadurch gekennzeichnet, dass** - in einem senkrecht zu der Längsachse (5) der Abdeckung (4) geführten Querschnitt der Abdeckung (4) betrachtet - zwischen einem von der inneren Mantelfläche (14) der Abdeckung (4) abgewandten Ende (16) der in ihrem unverformten Zustand vorliegenden Lasche (10) und einem der Lasche (10) zugewandten Ende (17) des Anschlags (11) ein in einer zu der Schwenkebene (8) parallelen Richtung gemessener Abstand (19) vorliegt, wobei vorzugsweise der Abstand (19) im Bereich zwischen 0,20 mm und 0,80 mm, vorzugsweise zwischen 0,25 mm und 0,70 mm, weiter vorzugsweise zwischen 0,30 mm und 0,60 mm, beträgt.

5. Schutzvorrichtung (1) nach einem der vorhergehenden Ansprüche, **dadurch gekennzeichnet, dass** sich das mindestens eine Führungselement (12, 13) ausgehend von der inneren Mantelfläche (14) der Abdeckung (4) in den Innenraum (6) erstreckt.

6. Schutzvorrichtung (1) nach einem der vorhergehenden Ansprüche, **gekennzeichnet durch** eine Mehrzahl von Führungselementen (12, 13), wobei vorzugsweise - in Längsrichtung der Abdeckung (4) betrachtet - mindestens ein Führungselement (12) vor dem Anschlag (10) und mindestens ein Führungselement (13) hinter dem Anschlag (10) angeordnet sind.

7. Schutzvorrichtung (1) nach einem der vorhergehenden Ansprüche, **dadurch gekennzeichnet, dass** die Abdeckung (4) in einem senkrecht zu ihrer Längsachse (5) geführten Querschnitt betrachtet u-förmig ausgebildet ist, wobei vorzugsweise eine Hochachse (20) des Querschnitts parallel zu der Schwenkebene (8) der Abdeckung (4) orientiert ist.

8. Schutzvorrichtung (1) nach einem der vorhergehenden Ansprüche, **dadurch gekennzeichnet, dass** die Basis (3) und die Abdeckung (4) bei Vorliegen der letzteren in ihrer Anfangsstellung mittels einer Sollbruchstelle (21) miteinander verbunden sind, die unter Aufbringung einer händischen Kraft zerstörbar ist, sodass die Abdeckung (4) nach der Zerstörung der Sollbruchstelle (21) bestimmungsgemäß in ihre Nutzstellung verschwenkbar ist.

9. Schutzvorrichtung (1) nach einem der vorhergehenden Ansprüche, **dadurch gekennzeichnet, dass** die Abdeckung (4) und die Basis (3) unter Ausbildung eines Folienscharniers (22) miteinander verbunden sind.

10. Schutzvorrichtung (1) nach einem der vorhergehenden Ansprüche, **dadurch gekennzeichnet, dass** die Abdeckung (4) und die Basis (3) bei Vorliegen in ihrer Anfangsstellung koaxial zueinander angeordnet sind, wobei vorzugsweise die Abdeckung (4) bei Vorliegen in ihrer Schutzstellung über die Anfangsstellung hinaus in die zweite Schwenkrichtung verschwenkt ist.

11. Schutzvorrichtung (1) nach einem der vorhergehenden Ansprüche, **gekennzeichnet durch** ein an der Basis (3) ausgebildetes Rastmittel (23), das dazu eingerichtet ist, formschlüssig mit einem Rastpartner (24) der Abdeckung (4) zusammenzuwirken, sodass die Abdeckung (4) in der Schutzstellung arretierbar ist.

12. Set umfassend eine Schutzvorrichtung (1) nach einem der vorherigen Ansprüche sowie einen Nadelträger (25) mit einem darin gelagerten Nadelrohr (2), wobei die Basis (3) der Schutzvorrichtung (1) formschlüssig mit dem Nadelträger (25) verbunden ist, sodass das Nadelrohr (2) bei Vorliegen der Abdeckung (4) in ihrer Anfangsstellung parallel zu der Längsachse (5) der Abdeckung (2) orientiert ist.

13. Set nach Anspruch 12, **dadurch gekennzeichnet, dass** - in einem senkrecht zu der Längsachse (5) der Abdeckung (4) geführten Querschnitt der Abdeckung (4) betrachtet - sich eine Laschenbasis (26) der Lasche (10), mit der die Lasche (10) an die innere Mantelfläche (14) der Abdeckung (4) angeschlossen ist, bei Vorliegen der Abdeckung (4) in ihrer Anfangsstellung sich mit dem Nadelrohr (2) in einer gemeinsamen, senkrecht zu der Schwenkebene (8) orientierten Ebene (27) befindet.

## Claims

1. A protective device (1) for a needle tube (2) of a syringe, the protective device comprising:
- a base (3); and
- a cover (4) connected to the base (3),
wherein the base (3) is configured to cooperate with a needle carrier (25) by positive form locking wherein the needle carrier (25) supports the needle tube,
wherein the cover (4) is configured elongated along a longitudinal axis (5) and defines an interior space (6) in which the needle tube (2) is storable so that a person is protected against an unintentional contact with the needle tube (2),
wherein the cover (4) is pivotably supported at the base (3) so that the cover (4) is transferable relative to the base (3) within a pivot plane (8) from a starting position where the needle tube (2) is stored in the interior (6) before being used for injection in a first pivot direction into an operating position where the needle tube (2) is available for injection, and eventually transferrable in a second pivot direction opposite to the first pivot direction into a protective position where the needle tube (2) is stored in the interior space (6) again after being used for injection,
wherein the cover (4) is open at one side in the pivot plane (8) so that the needle tube (2) is configured to initially exit from the interior space (6) for injection due to the pivot movement of the cover (4) and configured to enter the interior space (6) again after being used for the injection,
wherein the cover (4) includes a retaining device (9) in the interior space (6) wherein the retaining device is configured to interlock the needle tube (2) by positive form locking during transfer of the cover (4) into the protective position so that an unintentional exit of the needle tube (2) from the interior space (6) is prevented after a completed transfer of the cover (4) into the protective position,
wherein the retaining device (9) includes a deformable lobe (10), a stop (11) associated with the lobe (10) and at least one guide element (12, 13),
wherein the lobe (10) is configured to be deformed by the needle tube (2) during transfer of the cover (4) into its protective position and displaced in a first direction so that the needle tube (2) is able to enter into a safety area (7) arranged behind the lobe (10) in which the needle tube (2) is interlocked behind the lobe (10),
wherein the stop (11) is configured at the inner enveloping surface (14) of the cover (4) opposite from the lobe (10) so that the lobe (10) when deformed by the needle tube (2) contacts a contact surface (34) of the stop (11) in a second direction opposite to the first direction so that an unintentional exit of the needle tube (2) from the safety area (7) is prevented,
wherein the guide element (12, 13) is associated with the stop (11) and configured to guide the needle tube (2) so that a presence of the needle tube (2) below a stop surface (34) of the stop (11) is prevented after the needle tube (2) enters the safety area (7),
**characterized in that** the lobe (10) extends in undeformed condition from an inner enveloping surface (16) of the cover (4) into the interior space (6) and defines a lobe angle (15) of at least 35° with the pivot plane (8) viewed in a cross section orthogonal to the longitudinal axis (5) of the cover (4).

2. The protective device (1) according to claim 1, **characterized in that** the lobe angle (15) is at least 37.5°, advantageously at least 40° in the undeformed condition of the lobe (10).

3. The protective device (1) according to one of the preceding claims, **characterized in that** an offset (18) measured in a direction orthogonal to the pivot plane (8) is provided in a cross section of the cover (4) orthogonal to the longitudinal axis (5) of the cover (4) between an end (16) of the undeformed lobe (10) oriented away from the inner enveloping surface (14) of the cover (4) and an end (17) of the cover (11) oriented towards the lobe (10), wherein the offset (18) is advantageously in a range between 0.05 mm and 0.25 mm, advantageously between 0.10 mm and 0.20 mm.

4. The protective device (1) according to one of the preceding claims, **characterized in that** an offset (19) is provided in a cross section of the cover (4) orthogonal to the longitudinal axis (5) of the cover (4) between an end (16) of the undeformed lobe (10) oriented away from the inner enveloping surface (14) of the cover (4) and an end (17) of the stop (11) oriented towards the lobe (10) wherein the offset (19) is measured in a direction parallel to the pivot plane (8), wherein the offset (19) is advantageously in a range between 0.20 mm and 0.80 mm, advantageously between 0.25 mm and 0.70 mm, further advantageously between 0.30 mm and 0.60 mm.

5. The protective device (1) according to one of the preceding claims, **characterized in that** the at least one support element (12, 13) extends from the inner enveloping surface (14) of the cover (4) into the interior space (6).

6. The protective device (1) according to one of the preceding claims, **characterized by** a plurality of guide elements (12, 13), wherein advantageously at least one guide element (12) is arranged in front of the stop (10) and at least one guide element (13) is arranged behind the stop (10) viewed in the longitudinal direction of the cover (4).

7. The protective device (1) according to one of the preceding claims, **characterized in that** the cover (4) is configured U-shaped in a cross section orthogonal to its longitudinal axis (5) wherein a vertical axis (20) of the cross section is advantageously oriented parallel to the pivot plane (8) of the cover (4)

8. The protective device (1) according to one of the preceding claims, **characterized in that** the base (3) and the cover (4) are connected with one another by a rated fracture joint (21) when the cover (4) is in its starting position, wherein the rated fracture joint (21) is breakable by a hand force so that the cover (4) is pivotable into its operating position after breaking the rated fracture joint (21).

9. The protective device (1) according to one of the preceding claims, **characterized in that** the cover (4) and the base (3) are connected with one another by a film hinge (22).

10. The protective device (1) according to one of the preceding claims, **characterized in that** the cover (4) and the base (3) are arranged coaxial in their starting position, wherein the cover (4) is advantageously pivoted beyond the starting position in the second pivot direction when the cover (4) is in its protective position.

11. The protective device (1) according to one of the preceding claims, **characterized by** an interlocking device (23) configured at the base (3), wherein the interlocking device is configured to engage an interlocking partner (24) of the cover (4) by positive form locking so that the cover (4) is interlockable in the protective position.

12. A set including the protective device (1) according to one of the preceding claims, and a needle carrier (25) with a needle tube (2) supported therein, wherein the base (3) of the protective device (1) is connected with the needle carrier (25) by positive form locking, so that the needle tube (2) is oriented parallel to the longitudinal axis (5) of the needle tube (2) when the cover (4) is in its starting position.

13. The set according to claim 12, **characterized in that** a lobe base (26) of the lobe (10) that connects the lobe (10) at the inner enveloping surface (14) of the cover (4) is arranged in a common plane with the needle tube (2) that is perpendicular to the pivot plane (8) when the cover (4) is in its starting position viewed in a cross section of the cover (4) orthogonal to the longitudinal axis (5) of the cover (4).

## Revendications

1. Dispositif de protection (1) pour un tube d'aiguille (2) d'une seringue, comprenant
- une base (3) et
- un recouvrement (4) assemblé avec la base (3),
la base (3) étant configurée pour coopérer par complémentarité de forme avec un support d'aiguille (25) dans lequel est logé le tube d'aiguille (2),
le recouvrement (4) étant conçu de forme allongée le long d'un axe longitudinal (5) et délimitant un espace intérieur (6) dans lequel le tube d'aiguille (2) peut se loger, pour protéger une personne d'un contact intempestif avec le tube d'aiguille (2),
le recouvrement (4) étant logé de manière à pouvoir pivoter sur la base (3), de sorte qu'en partant d'une position initiale, dans laquelle, avant une utilisation pour une injection, le tube d'aiguille (2) est logé dans l'espace intérieur (6), le recouvrement (4) soit transférable à l'intérieur d'un plan de pivotement (8) par rapport à la base (3) dans une première direction de pivotement, dans une position d'utilisation, dans laquelle le tube d'aiguille (2) est libéré pour une utilisation pour une injection et finalement dans une deuxième direction de pivotement opposée à la première direction de pivotement, dans une position de protection, dans laquelle, après l'utilisation pour une injection, le tube d'aiguille (2) est de nouveau logé dans l'espace intérieur (6),
dans le plan de pivotement (8), le recouvrement (4) étant ouvert sur un côté, de sorte que suite aux déplacements en pivotement du recouvrement (4), pour l'utilisation pour une injection, le tube d'aiguille (2) puisse d'abord s'extraire de l'espace intérieur (6) et après l'utilisation pour une injection, puisse de nouveau pénétrer dans l'espace intérieur (6),
le recouvrement (4) comportant dans l'espace intérieur (6) un système de maintien (9), au moyen duquel, au fur et à mesure du transfert du recouvrement (4) dans la position de protection, le tube d'aiguille (2) est enclenchable par complémentarité de forme de telle sorte, qu'une fois que le transfert du recouvrement (4) dans la position de protection est effectué, une sortie intempestive du tube d'aiguille (2) hors de l'espace intérieur (6) soit entravé,
le système de maintien (9) comportant une patte (10) déformable, une butée (11) assignée à la patte (10), ainsi qu'au moins un élément de guidage (12, 13),
la patte (10) étant configurée pour, au fur et à mesure du transfert du recouvrement (4) dans sa position de protection, être déformée par le tube d'aiguille (2) et de ce fait, être refoulée dans une première direction, de telle sorte que le tube d'aiguille (2) puisse pénétrer dans une zone de sécurisation (7) de l'espace intérieur (6), située derrière la patte (10), dans laquelle le tube d'aiguille (2) est enclenché derrière la patte (10),
la butée (11) étant conçue à l'opposée de la patte (10) sur la surface enveloppante (14) intérieure du recouvrement (4) et étant configurée pour que, dans le cas de la déformation par le tube d'aiguille (2), la patte (10) tape dans une deuxième direction orientée à l'encontre de la première direction, contre une surface de butée (34) de la butée (11), suite à quoi, une extraction intempestive du tube d'aiguille (2) hors de la zone de sécurisation (7) est empêchée,
l'élément de guidage (12, 13) étant assigné à la butée (11) et étant configuré pour guider le tube d'aiguille (2) de telle sorte, qu'après sa pénétration dans la zone de sécurisation (7), un séjour du tube d'aiguille (2) en-dessous de la surface de butée (34) de la butée (11) soit empêché,
**caractérisé en ce que**
lorsqu'elle se présente dans un état non déformé, en partant d'une surface enveloppante (14) intérieure du recouvrement (4), la patte (10) s'étend dans l'espace intérieur (6) et (considérée par une section transversale guidée à la perpendiculaire de l'axe longitudinal (5) du recouvrement (4)), inclut avec le plan de pivotement (8) un angle de patte (15) d'au moins 35°.

2. Dispositif de protection (1) selon la revendication 1, **caractérisé en ce que** l'angle de patte (15) est d'au moins 37,5°, de préférence d'au moins 40°.

3. Dispositif de protection (1) selon l'une quelconque des revendications précédentes, **caractérisé en ce que** (considéré dans une section transversale du recouvrement (4) guidée à la perpendiculaire de l'axe longitudinal (5) du recouvrement (4)), entre une extrémité (16) opposée à la surface enveloppante (14) intérieure du recouvrement (4) de la patte (10) qui se présente dans son état non déformé et une extrémité (17) de la butée (11) qui fait face à la patte (10), il existe un écart (18) mesuré dans une direction perpendiculaire au plan de pivotement (8), de préférence l'écart (18) se situant dans l'ordre compris entre 0,05 mm et 0,25 mm, de préférence entre 0,10 mm et 0,20 mm.

4. Dispositif de protection (1) selon l'une quelconque des revendications précédentes, **caractérisé en ce que** (considéré dans une section transversale du recouvrement (4) guidée à la perpendiculaire de l'axe longitudinal (5) du recouvrement (4)), entre une extrémité (16) opposée à la surface enveloppante (14) intérieure du recouvrement (4) de la patte (10) qui se présente dans son état non déformé et une extrémité (17) de la butée (11) qui fait face à la patte (10), il existe un écart (19) mesuré dans la direction parallèle au plan de pivotement (8), de préférence l'écart (19) se situant dans l'ordre compris entre 0,20 mm et 0,80 mm, de préférence entre 0,25 mm et 0,70 mm, de manière plus préférentielle, entre 0,30 mm et 0,60 mm.

5. Dispositif de protection (1) selon l'une quelconque des revendications précédentes, **caractérisé en ce qu'**en partant de la surface enveloppante (14) intérieure du recouvrement (4), l'au moins un élément de guidage (12, 13) s'étend dans l'espace intérieur (6).

6. Dispositif de protection (1) selon l'une quelconque des revendications précédentes, **caractérisé par** une pluralité d'éléments de guidage (12, 13), de préférence (considérés dans la direction longitudinale du recouvrement (4)), au moins un élément de guidage (12) étant placé à l'avant de la butée (10) et au moins un élément de guidage (13) étant placé à l'arrière de la butée (10).

7. Dispositif de protection (1) selon l'une quelconque des revendications précédentes, **caractérisé en ce que** (considéré dans une section transversale guidée à la perpendiculaire de son axe longitudinal (5)), le recouvrement (4) est conçu en forme de U, de préférence un axe vertical (20) de la section transversale étant orienté à la parallèle du plan de pivotement (8) du recouvrement (4).

8. Dispositif de protection (1) selon l'une quelconque des revendications précédentes, **caractérisé en ce que** la base (3) et le recouvrement (4), lorsque ce dernier se présente dans sa position initiale sont assemblés l'un avec l'autre au moyen d'une zone de rupture théorique (21), qui est destructible par l'application d'une force manuelle, de sorte qu'après la destruction de la zone de rupture théorique (21), le recouvrement (4) soit susceptible d'être pivoté conformément à son usage dans sa position d'utilisation.

9. Dispositif de protection (1) selon l'une quelconque des revendications précédentes, **caractérisé en ce que** le recouvrement (4) et la base (3) sont assemblés l'un avec l'autre, en constituant une charnière pelliculaire (22).

10. Dispositif de protection (1) selon l'une quelconque des revendications précédentes, **caractérisé en ce que** lorsqu'ils se présentent dans leur position initiale, le recouvrement (4) et la base (3) sont placés de manière coaxiale, l'un par rapport à l'autre, lorsqu'il se trouve dans sa position de protection, le recouvrement (4) étant pivoté de préférence au-delà de la position initiale, dans la deuxième direction de pivotement.

11. Dispositif de protection (1) selon l'une quelconque des revendications précédentes, **caractérisé par** un moyen d'enclenchement (23) conçu sur la base (3), qui est configuré pour coopérer par complémentarité de forme avec un partenaire d'enclenchement (24) du recouvrement (4), de sorte que le recouvrement (4) puisse être bloqué dans la position de protection.

12. Kit, comprenant un dispositif de protection (1) selon l'une quelconque des revendications précédentes, ainsi qu'un support d'aiguille (25) doté d'un tube d'aiguille (2) logé dans celui-ci, la base (3) du dispositif de protection (1) étant assemblée par complémentarité de forme avec le support d'aiguille (25), de sorte que lorsque le recouvrement (4) se trouve dans sa position initiale, le tube d'aiguille (2) soit orienté à la parallèle de l'axe longitudinal (5) du recouvrement (2).

13. Kit selon la revendication 12, **caractérisé en ce que** (considérée dans une section transversale du recouvrement (4) guidée à la perpendiculaire de l'axe longitudinal (5) du recouvrement (4)), lorsque le recouvrement (4) se présente dans sa position initiale, une base (26) de patte de la patte (10), par laquelle la patte (10) est raccordée sur la surface enveloppante (14) intérieure du recouvrement (4) se trouve avec le tube d'aiguille (2) dans un plan (27) commun, orienté à la perpendiculaire du plan de pivotement (8).
